# EUROPEAN PATENT APPLICATION

(11) **EP 4 567 101 A1**
(43) Date of publication of application: **11.06.2025**
(21) Application number: 23850182.9
(22) Date of filing: 04.08.2023
(51) Int. Cl.: C12N 5/071, A23J 3/00, A23L 13/00, A23L 29/269, A23L 29/281, C12M 3/00, C12N 1/00

(54) **POROUS SCAFFOLD FOR CELL CULTURE AND PRODUCTION METHOD THEREOF**

(30) Priority: 04.08.2022 JP 2022124446
(71) Applicant: San-Ei Gen F.F.I., INC., Toyonaka-shi, Osaka 561-8588 (JP); Integriculture Inc., Tokyo 113-0033 (JP)
(72) Inventor: HATANO, Hiroaki, Tokyo 162-8666 (JP); LU, Mengxue, Tokyo 162-8666 (JP); ONISHI, Masanobu, Toyonaka-shi, Osaka 561-8588 (JP); MAEDA, Kazuhiro, Toyonaka-shi, Osaka 561-8588 (JP); ONODERA, Makoto, Toyonaka-shi, Osaka 561-8588 (JP)
(74) Representative: Bryn Aarflot AS
(86) International application number: PCT/JP2023/028625
(87) International publication number: WO 2024/029629

(57) **Abstract**

The object of the present invention is to provide a porous scaffold for cell culture which does not deteriorate even when subjected to sterilization treatment such as autoclaving, and is capable of culturing various cells; and a production method thereof.

The present invention is a porous scaffold for cell culture comprising a crosslinked aggregate of gelatin and xanthan gum or an analog thereof, wherein the shape of the porous scaffold for cell culture is not changed during heat sterilization; and a method of producing a porous scaffold for cell culture, comprising: a mixing and foaming step of mixing and foaming gelatin and xanthan gum or an analog thereof; and a crosslinking step of crosslinking gelatin and xanthan gum or an analog thereof in the obtained foam.

## Description

### TECHNICAL FIELD

The present invention relates to a porous scaffold for cell culture and a production method thereof. Furthermore, the present invention relates to a food composition in which edible cultured cells are attached to a porous scaffold for cell culture, and a production method thereof.

### BACKGROUND ART

Biological tissues are generally composed of cells and extracellular matrix produced by the cells themselves. The extracellular matrix exists surrounding cells. Its main components are structural proteins (collagen, elastin, keratin, etc.), glycosaminoglycans (hyaluronic acid, chondroitin sulfate, etc.), and cell adhesion molecules (fibronectin, laminin, fibrinogen, etc.). The extracellular matrix plays an important role as a scaffold for cell activities such as cell adhesion, proliferation and differentiation.

In cell culture, a scaffold which replaces the extracellular matrix is also necessary as a structural support and for cell adhesion, proliferation, differentiation, etc. The role of activation of cell activity, provision of sufficient nutrients to cells, and excretion of waste is required to a scaffold for cell culture, so a form of porous bodies or highly water-containing gel is preferred. Furthermore, biodegradable scaffolds have also attracted attention as a scaffold for regenerative medicine and regenerated tissues, which have been actively researched in recent years.

The mainly used biodegradable scaffolds include synthetic polymer materials and natural polymer materials. The synthetic polymer materials include, for example, polyglycolic acid (PGA), polylactic acid (PLA), a copolymer of polyglycolic acid and polylactic acid (PLGA), polyethylene glycol (PEG), polycaprolactone, and the like. Natural polymeric materials include, for example, components of extracellular matrix, and specifically include collagen, gelatin, alginic acid, hyaluronic acid, agarose, chitosan, fibrin, fibroin, and the like. Synthetic polymer materials generally have high mechanical strength and excellent function as a structural support, but they have a problem of low affinity with cells. Natural polymer materials generally have high cell affinity, but have problems of low mechanical strength and easy deformation (Non-Patent Literature 1). As described above, both synthetic polymer materials and natural polymer materials have had problems in either cell adhesion or mechanical strength.

Collagen adhesion proteins such as integrin are expressed on the surface of many cells, and collagen has extremely strong adhesion to various cells. Therefore, scaffolds using natural polymeric materials such as collagen, which mimic the extracellular matrix, are attracting attention. However, as mentioned above, natural polymer materials have the problems of low mechanical strength and easy deformation and changing their shapes during heat sterilization. There was a need for a scaffold with improved mechanical strength, which shape does not change during heat sterilization, while keeping the characteristics of natural polymer materials.

Patent Literature 1 describes a scaffold material for tissue regeneration which consists of a water-insoluble hydrogel in which collagen, gelatin or another protein molecule is cross-linked with (-)-epigallocatechin gallate or another polyphenol, or a sponge obtained by drying the water-insoluble hydrogel. Patent Literature 2 describes a scaffold for vascular endothelial cell migration which comprises genetically modified gelatin having an amino acid sequence derived from a partial amino acid sequence of collagen.

However, Patent Literatures 1 or 2 does not describe whether the scaffold of Patent Literatures 1 or 2 does not deteriorate even when subjected to sterilization treatment such as autoclaving, and is capable of culturing various cells. Further, Patent Literatures 1 or 2 does not describe at all a scaffold in which gelatin and xanthan gum or the like are combined. As in the test of Comparative example 2 described below, the scaffold containing only gelatin did not have sufficient cell adhesion and proliferation.

Non-Patent Literatures 2 and 3 describe production of porous scaffolds by stabilizing gelatin with a crosslinking agent such as glutaraldehyde using a bubble method. However, the present inventors verified the production, but were unable to produce these stable porous scaffolds.

Furthermore, the scaffolds of Non-Patent Literatures 2 and 3 have a denaturation temperature of 73.5 to 87.6°C (Table 1 of Non-Patent Literature 2, Table 2 of Non-Patent Literature 3), and will deteriorate when subjected to sterilization treatment such as autoclaving. Non-Patent Literatures 2 or 3 does not describe at all a scaffold wherein gelatin and xanthan gum or the like are combined. As in the test of Comparative example 2 described below, the scaffold containing only gelatin did not have sufficient cell adhesion and proliferation.

Furthermore, in recent years, the world's food supply and demand have been discussed, as the world's population has increased. For example, the world population in 2050 is expected to increase approximately 1.3 times compared to those in 2010 to reach approximately 8.64 billion people. The world's agricultural land area in 2050 is expected to increase by about 70 million hectares to about 1.61 billion hectares due to the average temperature rise by approximately 2% by global warming. Thus, the projected growth rate of the world's population exceeds the projected growth rate of the world's agricultural land area.

Therefore, it has been expected to develop new foods, for example, new foods that utilize other means such as culture technology. For example, cultured meat is a food product produced by cell culture using tissue engineering technology. Research and development of cultured meat has been progressing in recent years at various research institutes, universities, and companies. Cultured meat technology is highly efficient in food production compared to livestock farming, which requires large amounts of feed for animals to consume and vast farmland necessary for that. Cultured meat technology can eliminate the effects of animal infectious diseases or the like that can cause problems in livestock farming, as well as the effects of antibiotics or the like administered to animals. In addition, it is possible to eliminate the influence of bacteria or viruses that may contaminate livestock foods by producing in a sterile environment.

Patent Literature 3 describes an edible composition that comprises a three-dimensional porous scaffold, myotubes containing myotube nuclei, and a plurality of cell types. The three-dimensional porous scaffold includes textured proteins, non-textured proteins, polysaccharides, and the like. Textured soy protein was used in the Examples. However, when the present inventors cultured cells using soybean protein, the cultured cells did not proliferate well, and it was not possible to produce a food composition containing the cultured cells.

Patent Literature 4 describes an edible dehydrated food comprising cultured animal muscle cells in combination with a plant-derived hydrogel. Edible microcarriers on which cultured cells are grown can be used as a plant-derived hydrogel. Polysaccharides such as pectin and polypeptides such as calcidone have been described as the edible microcarrier. However, no specific examples are described.

### CITATION LIST

### PATENT LITERATURE

Patent Literature 1: JP 2008-125916
Patent Literature 2: JP 2013-074936
Patent Literature 3: JP 2020-527054
Patent Literature 4: JP 2017-505138

### NON-PATENT LITERATURE

Non-Patent Literature 1: Journal of the Society of Materials Science, Japan, 2014, Vol. 63, No. 9, 684-689
Non-Patent Literature 2: Materials Science and Engineering C, 48 (2015) 63-70
Non-Patent Literature 3: Materials Science and Engineering C, 63 (2016) 1-9

### SUMMARY OF THE INVENTION

### TECHNICAL PROBLEM

An object of the present invention is to provide a porous scaffold for cell culture which does not deteriorate even when subjected to sterilization treatment such as autoclaving, and is capable of culturing various cells; and a production method thereof. Another object of the present invention is to provide a food composition in which edible cultured cells are attached to a porous scaffold for cell culture, and a production method thereof.

In addition, another object of the present invention is to provide a scaffold on which even cell lines can proliferate suitably, which cell lines have poor proliferative properties on known scaffolds made of PET resin (for example, Comparative example 1) or porous scaffolds made of gelatin fiber-based material (for example, Comparative example 2).

### SOLUTION TO THE PROBLEM

As a result of intensive studies aimed at solving the problems of the present invention described above, the inventors of the present application have surprisingly discovered that a porous scaffold for cell culture comprising a crosslinked aggregate prepared by mixing and crosslinking gelatin and xanthan gum preserves the properties of collagen or gelatin such as cell adhesion, proliferation and differentiation; the mechanical strength which collagen or gelatin lacks is greatly improved; its shape does not change; and a variety of cells can be cultured without deterioration even when the porous scaffold for cell culture are subjected to sterilization treatment such as autoclaving. Accordingly, the present invention was accomplished.

That is, the present invention is as follows.
[1] A porous scaffold for cell culture comprising a crosslinked aggregate of gelatin and xanthan gum or an analog thereof, wherein the shape of the porous scaffold for cell culture is not changed during heat sterilization.
[2] The porous scaffold for cell culture according to [1], wherein the crosslinked aggregate further comprises insoluble cellulose.
[3] The porous scaffold for cell culture according to [2], wherein the insoluble cellulose is fermentation-derived cellulose, citrus fiber, or microcrystalline cellulose.
[4] A method of producing a porous scaffold for cell culture, comprising:
   a mixing and foaming step of mixing and foaming gelatin and xanthan gum or an analog thereof; and
   a crosslinking step of crosslinking gelatin and xanthan gum or an analog thereof in the obtained foam.
[5] The production method according to [4], further comprising a drying step of drying the product obtained in the crosslinking step.
[6] The production method according to [4] or [5], wherein in the mixing and foaming step, insoluble cellulose is further added to gelatin and xanthan gum or an analog thereof, and the obtained mixture is foamed.
[7] The production method according to any one of [4] to [6], wherein in the crosslinking step, crosslinking is performed by enzyme treatment and/or heat treatment.
[8] A porous scaffold for cell culture produced by the production method according to any one of [4] to [7].
[9] A food composition in which edible cultured cells are attached to a porous scaffold for cell culture according to any one of [1] to [3] and [8].
[10] A method of producing a food composition in which edible cultured cells are attached to a porous scaffold for cell culture according to any one of [1] to [3] and [8], comprising a culture step of culturing the edible cells together with the porous scaffold for cell culture.
[11] The production method according to [10], further comprising a differentiation step of differentiating the edible cultured cells.

### ADVANTAGEOUS EFFECTS OF THE INVENTION

The present invention can provide a porous scaffold for cell culture which is edible, has greatly improved mechanical strength, does not change its shape, does not deteriorate even after sterilization treatment such as autoclaving, and is capable of culturing various cells; and a production method thereof. Furthermore, the present invention can provide a food composition in which edible cultured cells are attached to a porous scaffold for cell culture, and a production method thereof.

### BRIEF DESCRIPTION OF THE DRAWINGS

FIG. 1 is a diagram showing evaluation of adhesive ability and proliferation ability of the porous scaffold for cell culture using HEK293T cells in Test example 1.
FIG. 2 is a diagram showing evaluation of adhesive ability and proliferation ability of the porous scaffold for cell culture using duck liver-derived primary cells in Test example 2.
FIG. 3 is a diagram showing evaluation of adhesive ability and proliferation ability of the porous scaffold for cell culture using duck liver-derived primary cells in Test example 3.
FIG. 4 is a diagram showing evaluation of adhesive ability and proliferation ability of the porous scaffold for cell culture using duck liver-derived primary cells in Test example 3.
FIG. 5 is a diagram showing evaluation of adhesive ability and proliferation of a porous scaffold for cell culture using chicken muscle-derived primary cells in Test example 4.
FIG. 6 is a diagram showing evaluation of adhesive ability and proliferation ability of the porous scaffold for cell culture using chicken muscle-derived primary cells in Test example 4.
FIG. 7 is a diagram showing evaluation of adhesive ability and proliferation ability of the porous scaffold for cell culture using duck liver-derived primary cells in Test example 5.
FIG. 8 is a diagram showing evaluation of adhesive ability and proliferation ability of RL34 cells (rat-derived liver epithelial cells) using the PET resin scaffold of Comparative example 1, the gelatin fiber-based material porous scaffold of Comparative example 2 and the porous scaffold for cell culture of Example 5 in Test example 6.
FIG. 9 is a diagram showing evaluation of adhesive ability and proliferation ability of the porous scaffold for cell culture of Example 5 using various established cell lines and bovine-derived primary cells in Test example 6.
FIG. 10 is a diagram showing evaluation of adhesive ability and proliferation ability of the porous scaffold for cell culture of Example 5 using various established cell lines and bovine-derived primary cells in Test example 6.
FIG. 11 is an image showing the shape of the porous scaffold obtained after the pulverizing step in Test example 7.
FIG. 12 is a diagram showing a shape comparison evaluation of the porous scaffold for cell culture using duck liver-derived primary cells in Test example 7.

### DESCRIPTION OF THE INVENTION

### 1. Porous scaffold for cell culture

The present invention relates to a porous scaffold for cell culture comprising a crosslinked aggregate of gelatin and xanthan gum or an analog thereof, wherein the shape of the porous scaffold for cell culture is not changed during heat sterilization. A crosslinked aggregate prepared by mixing and crosslinking gelatin and xanthan gum or the like is one produced by preparing aggregates by electrostatic interaction (ionic bond, hydrogen bond) between gelatin and xanthan gum or the like, and also intertwining the network-structured polymer networks formed by association of respective gelatin and xanthan gum or the like with each other; and by further crosslinking the obtained aggregates. It is believed that this greatly improves the mechanical strength of the porous scaffold for cell culture.

### <Gelatin>

As the gelatin used in the present invention, gelatin obtained, for example, by extracting collagen with hot water and then hydrolyzing the extracted collagen by alkali treatment, acid treatment, enzyme treatment, etc. while using a collagen-containing raw material such as a bone and skin of a mammal such as cow and pig, and a bone, skin and scale of fish such as shark and tilapia, can be used. The alkali includes, for example, sodium hydroxide, calcium hydroxide and the like. The acid includes, for example, hydrochloric acid, sulfuric acid, nitric acid and the like. The enzyme may be any enzyme as long as the enzyme has the function of cleaving peptide bonds in gelatin. The enzyme is usually an enzyme called a protease. Specifically the enzyme includes, for example, collagenase, thiol protease, serine protease, acidic protease, alkaline protease, metal protease and the like, and these can be used alone or in combination. The thiol protease includes, for example, plant-derived chymopapain, papain, promelain, ficin, animal-derived cathepsin, calcium-dependent protease and the like. The serine protease includes trypsin, cathepsin D and the like. The acidic protease includes pepsin, chymosin and the like.

The gelatin that can be used in the present invention includes gelatin hydrolyzed by acid treatment or alkali treatment, gelatin hydrolyzed by enzyme treatment, and the like. Any gelatin may be used, but in the present invention it is desirable to charge the gelatin positively, so it is necessary to consider the isoelectric point (pl value) of each type of gelatin. For example, gelatin hydrolyzed by acid treatment or enzyme treatment has a pl value of 7 to 9, which is close to that of collagen, because deamidation progresses less during hydrolysis compared to gelatin hydrolyzed by base treatment. The pI value of alkali-treated gelatin is 5. Gelatin becomes positively charged by adjusting the pH to a value lower than these pl values. Taking into account that enzyme treatment may be performed in the crosslinking step of crosslinking gelatin and xanthan gum, gelatin hydrolyzed by acid treatment which is easy to use in an acidic range (pH 5 to 6.5) at which pH enzymes are more likely to function, is more preferably used. Appropriate adjustment of pH during the production process strengthens the electrostatic interaction with xanthan gum or an analog thereof, contributing to the mechanical strength and hardness of the porous scaffold for cell culture of the present invention.

The weight average molecular weight of gelatin that can be used in the present invention is, for example, about 5,000 to about 20,000, preferably about 8,000 to about 15,000, about 10,000 to about 17,000, about 12,000 to about 18,000, about 15,000 to 20,000, etc. By selecting the weight average molecular weight of gelatin or by selecting the type of raw material of gelatin, the mechanical strength and hardness of the porous scaffold for cell culture of the present invention can be adjusted to a desired range.

### <Xanthan gum or an analog thereof>

Xanthan gum used in the present invention is a polysaccharide produced extracellularly by Xanthomonas campestris. For example, xanthan gum in the form of potassium salt, sodium salt, calcium salt, etc. can be used depending on the type of salts used in the production method of xanthan gum. A preferred weight average molecular weight of xanthan gum is, for example, about 2 million to about 50 million. Xanthan gum usually consists of repeating units of two molecules of glucose, two molecules of mannose, and glucuronic acid. Since xanthan gum has an acidic group, xanthan gum has a strong electrostatic interaction with gelatin, especially with gelatin hydrolyzed by acid treatment or enzyme treatment, whose isoionic point is pH 7 to 9. The strong electrostatic interaction contributes to the mechanical strength and hardness of the porous scaffold for cell culture. The analog of xanthan gum includes, for example, a xanthan gum derivative which can form an aggregate with gelatin similarly to xanthan gum.

Xanthan gum derivatives include, for example, deacetylated xanthan gum, low acetylated xanthan gum, low pyruvate xanthan gum, cationized xanthan gum, crosslinked xanthan gum, acid-treated xanthan gum and the like.

The content of xanthan gum or an analog thereof in the porous scaffold for cell culture of the present invention is, for example, about 1 to about 15% by mass, preferably about 2 to about 10% by mass, more preferably about 3 to about 8% by mass, based on gelatin.

When gellan gum or sodium alginate was used instead of xanthan gum, etc., the mechanical strength and hardness of the obtained porous scaffold for cell culture were not sufficiently improved.

### <Insoluble cellulose>

The crosslinked aggregate in the porous scaffold for cell culture of the present invention can further comprises insoluble cellulose within a range of the amount which does not inhibit the effect of the porous scaffold for cell culture of the present invention. The insoluble cellulose includes, for example, fermentation-derived cellulose, citrus fiber, microcrystalline cellulose, and a mixture thereof. Preferred insoluble celluloses include, for example, fermentation-derived cellulose, citrus fibers, and microcrystalline cellulose. For example, by including insoluble cellulose such as fermentation-derived cellulose, the bubbles prepared in the production of the porous scaffold for cell culture of the present invention can be more stabilized, and the mechanical strength of the porous scaffold for cell culture can be further increased. The improvement of mechanical strength is believed to be due to the fact that the polymer networks formed by the association of gelatin and xanthan gum, etc. respectively, intertwines with each other and further with the insoluble cellulose.

The content of insoluble cellulose varies depending on the type of insoluble cellulose, but for example, it is about 0.1 to about 30% by mass, preferably about 0.2 to about 20% by mass, more preferably about 0.3 to about 10% by mass, based on gelatin. When the insoluble cellulose is fermentation-derived cellulose, the content of the insoluble cellulose is, for example, about 0.1 to about 15% by mass, preferably about 0.2 to about 12% by mass, more preferably about 0.4 to about 10% by mass, based on gelatin.

### <Other components>

The porous scaffold for cell culture of the present invention can further comprise other components within a range of the amount which does not inhibit the effects of the porous scaffold for cell culture of the present invention. The other components include, for example, a polysaccharide thickener, protein decomposition product, and the like. The polysaccharide thickener includes, for example, deacylated gellan gum (and an alkaline earth metal salt), white wood ear polysaccharide, agar, mannan, hyaluronic acid, carrageenan, modified cellulose such as sodium carboxymethyl cellulose (CMC-Na) and hydroxypropyl methylcellulose (HPMC), and the mixture thereof, and the like. The protein decomposition product includes, for example, wheat protein decomposition product, soybean protein decomposition product, collagen peptide, and a mixture thereof, and the like. The content of the polysaccharide thickener or protein decomposition product varies depending on the type of polysaccharide thickener or protein decomposition product, but it is, for example, about 0.1 to about 30% by mass, preferably about 0.2 to about 20% by mass, more preferably about 0.3 to about 10% by mass, based on gelatin.

### <Coating agent>

The porous scaffold for cell culture of the present invention can also be further coated with a coating agent. When coated with a coating agent, the cultured cells and the porous scaffold are preferably attached to each other via the coating agent. The coating agent includes, for example, a coating agent made of a polypeptide or protein such as polylysine, RGD peptide-containing polypeptide, fibronectin, laminin and fibrinogen, and hyaluronic acid, chitin, chitosan, chondroitin sulfate and a mixture thereof, and the like. Preferred coating agent includes polylysine, and the like. By coating with a coating agent, adhesion of cells to the porous scaffold of edible polysaccharide can be adjusted and cell proliferation can be promoted.

The content of the coating agent is, for example, about 1 to 50% by mass, preferably about 2 to 20% by mass, more preferably about 3 to 10% by mass, based on the porous scaffold for cell culture.

In the porous scaffold for cell culture of the present invention, the constituent components are mutually crosslinked, preferably the constituent components are mutually crosslinked by enzyme treatment and/or heat treatment. Enzyme treatment and heat treatment will be explained below.

The porous scaffold for cell culture of the present invention is characterized by having a porous structure, and the pore size thereof is, for example, about 10 µm to about 1 mm.

In the present invention, "the shape is not changed during heat sterilization" means that the shape of the porous scaffold for cell culture is not changed when heat sterilized, for example, by high-pressure steam sterilization (autoclaving). Specifically, this means that the scaffold structure is not dissolved and the porous structure does not collapse under the condition used in Examples, which is "autoclaving sterilization at 120°C for 20 minutes."

### <Shape of the porous scaffold for cell culture of the present invention>

The prepared porous scaffold for cell culture of the present invention is preferably used after being molded or cut into a shape suitable for use in cell culture. The shape includes, for example, rectangular, spherical, cylindrical, beads, fine powder and the like. The size of the prepared porous scaffold for cell culture includes a size suitable for the culture apparatus used, and the major axis is, for example, 0.5 to 20 mm, preferably 1 to 10 mm.

The porous scaffold for cell culture of the present invention is characterized by maintaining its certain structure even when pulverized. For the purpose of increasing the specific surface area of the scaffold, the scaffold can also be appropriately pulverized and processed into fine powders. The particle size of the scaffold after pulverization is, for example, 20 to 50 µm, 50 to 100 µm, 100 µm to 2 mm, or the like.

Furthermore, even if a scaffold has the same composition as the porous scaffold for cell culture of the present invention and is processed into fine powders without being processed into a porous structure, the effects of the present invention can be achieved.

### 2. Method of producing the porous scaffold for cell culture

The porous scaffold for cell culture of the present invention may be produced by a method comprising:
a mixing and foaming step of mixing and foaming gelatin and xanthan gum or an analog thereof; and
a crosslinking step of crosslinking gelatin and xanthan gum or an analog thereof in the obtained foam.

### <Mixing and foaming step>

In the mixing and foaming step, gelatin and xanthan gum or an analog thereof are first mixed in water. Specifically, possible methods include a method of preparing an aqueous solution of gelatin and an aqueous solution of xanthan gum, etc. and then mixing both aqueous solutions; a method of preparing an aqueous solution of xanthan gum, etc. and then adding gelatin to the aqueous solution and dissolving it; and a method of mixing gelatin and xanthan gum, etc. in the powder form, and dissolving the mixture in water. Xanthan gum is highly easy to be hydrated and tends to clump when dissolved in water. Furthermore, in order to avoid coagulation of gelatin and xanthan gum, etc. before they are dissolved in water, it is preferable to prepare an aqueous solution of gelatin and an aqueous solution of xanthan gum, etc., respectively, and then mix the two aqueous solutions. Insoluble cellulose, other components, and the like may be further mixed into the aqueous solution of gelatin and xanthan gum or an analog thereof. For example, by including insoluble cellulose such as fermentation-derived cellulose, the bubbles can be further stabilized, and the mechanical strength of the porous scaffold for cell culture is further improved.

The concentration of gelatin in water includes, for example, about 1 to about 10% by mass, preferably about 2 to about 8% by mass, and more preferably about 3 to about 7% by mass. The amounts of xanthan gum or an analog thereof, acid, and polysaccharide thickener to be added are as described above. The mixing temperature includes, for example, about 40 to about 95°C, preferably about 60 to about 90°C, more preferably about 70 to about 85°C.

More preferably, an acid is added in the mixing and foaming step to adjust pH of the mixture to be below the isoelectric point of gelatin (for acid-treated gelatin and enzyme-treated gelatin: pl value 7 to 9; for alkali-treated gelatin: pl value 5). Without being bound by the mechanism described below, by adding an acid during scaffold production to lower the pH below the isoelectric point of gelatin, the gelatin is positively charged and can strongly electrostatically interacts with the negatively charged xanthan gum. In this state, by further performing the following crosslinking step of enzyme treatment and/or heat treatment, crosslinking between gelatin and xanthan gum is promoted, and the mechanical strength of the porous scaffold for cell culture is improved.

The acid includes, for example, citric acid, oxalic acid, maleic acid, malic acid, hydrochloric acid, sulfuric acid, phosphoric acid, methanesulfonic acid, and the like. Preferred acids include citric acid, oxalic acid, maleic acid, malic acid, hydrochloric acid, sulfuric acid, phosphoric acid, methanesulfonic acid and the like, and more preferably citric acid and the like. The content of the acid varies depending on the type and content of the constituent components of the porous scaffold for cell culture, but it may be, for example, about 0.1 to about 2% by mass, preferably about 0.2% to about 1.5% by mass, more preferably from about 0.4 to about 1% by mass, based on gelatin. Further, the pH of the scaffold material after addition of the acid is, for example, pH 3 to 6, preferably pH 3 to 5.

Subsequently, the resulting mixture is foamed to prepare a foam. The foaming means are not particularly limited, and any means may be used as long as it can form foams of about 10 µm to about 1 mm or the like, for example. For example, the mixture can be vigorously stirred using a mixer or the like so as to incorporate air. Further, a foam can also be prepared by blowing generated bubbles using a bubble generator such as a micro valve.

### <Crosslinking step>

Gelatin and xanthan gum or an analog thereof are crosslinked in the obtained foam by a desired method. The crosslinking methods include, for example, enzyme treatment and/or heat treatment.

The enzyme that can be used in the enzyme treatment includes, for example, transglutaminase, laccase, peroxidase, lysyl oxidase, protein disulfide isomerase, protein disulfide reductase, sulfhydryl oxidase, lipoxygenase, polyphenol oxidase (tyrosinase) and the like, and preferably transglutaminase and the like. The enzyme treatment can be carried out by a conventionally known method depending on the enzyme used. When the enzyme is transglutaminase, the enzyme reaction can be carried out at 40°C for 1 hour, for example. In addition, considering the optimum reaction pH of the enzyme used in the enzyme treatment, the pH is adjusted to, for example, 4 to 7. Further, if salts are necessary for the enzyme to act stably, salts may be added as necessary. The enzyme treatment promotes insolubilization of gelatin and improves the shape retention of the porous scaffolds for cell culture.

When the polysaccharide thickener is gelled by adding an alkaline earth metal salt, the alkaline earth metal salt is added to gel the polysaccharide thickener in the crosslinking step.

Further, when crosslinking is strengthened by heat treatment, there are no particular limitations, but it can be carried out, for example, at about 150 to about 210°C, preferably about 160 to about 200°C, for about 1 to 10 minutes. Crosslinking by heat treatment greatly improves the mechanical strength of the porous scaffold for cell culture, allowing it to withstand autoclaving sterilization.

### <Drying step>

It is possible to use the product obtained in the crosslinking step as a scaffold for cell culture, but when considering labor in transportation or the like, it may be better to dry the product. In this case, drying step may be performed following the crosslinking step. The drying step that can be used in the present invention includes, for example, dry heat drying, reduced pressure drying, freeze drying and the like.

Furthermore, when the scaffold is desired to be sterilized (e.g., autoclaving, etc.) before use for cell culture, it is desirable to further strengthen the crosslinking between gelatin and xanthan gum or an analog thereof. In this case, the product may preferably be treated by dry heat drying.

Dry heat drying is not particularly limited, but can be carried out, for example, under normal pressure at about 40 to about 90°C, preferably about 50 to about 70°C until dry. By drying, a spongy porous product can be obtained.

Furthermore, by further performing a heat treatment following the drying step, the crosslinking between gelatin and xanthan gum or an analog thereof can be strengthened. The heat treatment is not particularly limited, but can be carried out, for example, at about 150 to about 210°C, preferably about 160 to about 200°C, for about 1 to 10 minutes. Crosslinking by the heat treatment greatly improves the mechanical strength of the porous scaffold for cell culture.

The obtained porous scaffold for cell culture is shaped or cut into a scaffold in a shape used for cell culture. The shape is as described above. Furthermore, if necessary, the porous scaffold for cell culture can be coated with a coating agent.

### <Pulverizing step>

As mentioned above, the porous scaffold for cell culture of the present invention may be appropriately subjected to pulverizing step in order to improve the specific surface area. Specifically, it can be easily prepared by finely pulverizing, shearing and cutting the scaffold with a shearing device and passing it through a mesh.

The produced porous scaffold for cell culture can be sterilized before use. The sterilization process is not particularly limited, but can be sterilized by, for example, ethylene oxide gas sterilization, radiation sterilization, high pressure steam sterilization (autoclaving), dry sterilization, or the like.

### 3. Food composition

The food composition of the present invention is a food composition in which edible cultured cells are attached to the above porous scaffold for cell culture.

Since the constituent components of the porous scaffold for cell culture of the present invention are edible, the food composition in which edible cultured cells are attached to the porous scaffold for cell culture can be eaten as a food.

### <Edible cells>

The edible cells to be cultured are generally those of animals other than human. The cells may be, for example, cells from a mammal such as cow, pig, sheep, goat, deer, boar, horse, rabbit, bear and whale; a bird such as chicken, duck, turkey, goose, ostrich, pheasant and pigeon; a reptile such as crocodile and alligator; a fish such as tuna, salmon, mackerel, sardines, anchovies, perch, catfish, carp, cod, eel, flounder, puffer fish, grouper, halibut, herring, dolphinfish, marlin, pike, shark, snapper, flounder, swordfish, tilapia and trout; a crustacean such as crab, crayfish, lobster, shrimp and small shrimp; a mollusc such as abalone, clam, oyster, scallop and snail; a cephalopod such as cuttlefish, octopus and squid; an insect such as cricket, grasshopper and mealworm (larvae of mealworm); and the like.

The edible cultured cells may be cells from various organs and tissues of the above animals. The organs and tissues include, for example, skeletal muscle, smooth muscle, heart, liver, kidney, stomach, intestine, and the like. Specifically, the cells include myoblasts, satellite cells, adipocytes, endothelial cells, hepatic parenchymal cells, hepatic nonparenchymal cells, renal cells and the like. Cells obtained by further differentiation of cultured cells such as myofibrils, are also included in the cultured cells in the present invention.

The food composition of the present invention may be produced by the production method described below.

### 4. Method of producing a food composition

The food composition of the present invention may be produced by a production method which comprises a culturing step of culturing edible cells together with the above porous scaffold for cell culture.

### <Culturing step>

For culturing the cells, a commonly used culturing method suitable for respective cells is used. Cells are cultured in a suitable medium with a porous scaffold for cell culture. As the appropriate medium, it is preferable to select a medium suitable for the cells used.

The cultured cells may also be further differentiated into, for example, myofibrils or the like.

A mixture produced by culturing cells with a porous scaffold for cell culture may be used as a food composition by removing the medium and washing as necessary. It may be washed by pelleting it by centrifugation or the like and rinsing it.

It is also possible to add a gelling agent, a thickener or the like to the obtained mixture and to process and mold the mixture into a paste or gel form to make a food composition, or to heat and freeze the mixture to make a food composition.

### EXAMPLE

The present invention is explained below using Examples, Comparative examples, Test examples, etc., but the present invention is not limited to these Examples.

### Examples 1 to 19

### Preparation of porous scaffold for cell culture

Gelatin in the amount described in Tables 1 to 3 was stirred and dissolved in 50 parts by mass of ion-exchanged water while stirring at 80°C. Xanthan gum and polysaccharide thickener (excluding calcium chloride, in Example 3) in the amount described in Tables 1 to 3, and optionally insoluble cellulose and other ingredients were stirred and dissolved in 50 parts by mass of ion-exchanged water while stirring at 80°C. Next, while stirring the prepared aqueous solution of xanthan gum and polysaccharide thickener and the others, the dissolved aqueous gelatin solution was added thereto, and then acid was added, and the final concentrations of the components after mixing the solutions were adjusted to those shown in Tables 1 to 3, and the obtained mixture was stirred. Subsequently, the obtained composition was foamed with a hand mixer. A solution of the enzyme and calcium chloride (in Example 3) dissolved in a small amount of ion-exchanged water was added to the foamed composition and mixed with a hand mixer until uniform. The obtained foamed composition was added to a stainless steel pad to a thickness of about 1 cm and molded. The stainless steel pad was placed in a constant temperature layer at 40°C and kept warm for 1 hour. The obtained foamed composition was dried in the constant temperature layer at 60°C for about 15 hours. Furthermore, heat treatment was performed in an oven at 180°C for 7 minutes. Porous scaffolds for cell culture were prepared by cutting into a size of 5 mm x 5 mm.

In Test examples 1 to 4, the porous scaffolds for cell culture were immersed in an aqueous solution containing 0.5% by mass of calcium chloride and sterilized in an autoclave at 120°C for 20 minutes before use for cell culture.

In Test examples 5 and 6, the porous scaffolds for cell culture were immersed in ion-exchanged water and sterilized in an autoclave at 121°C for 20 minutes before use for cell culture.

Even after autoclaving, the mechanical strength and porous structure (pore size: about 100 to about 1000 µm) were maintained. Furthermore, even when 40 times the mass of ion-exchanged water relative to the dry mass of the porous scaffold for cell culture was added to the porous scaffold for cell culture, the structure of the porous scaffold for cell culture was maintained.

**[Table 1]**

| (Parts by mass) | | Example 1 | Example 2 | Example 3 | Example 4 | Example 5 | Example 6 |
|---|---|---|---|---|---|---|---|
| Gelatin | Amount | 5 | 5 | 5 | 5 | 5 | 5 |
| | Kind | P-203 | P-203 | P-203 | P-203 | P-204 | P-203 |
| Xanthan gum | Amount | 0.2 | 0.3 | 0.3 | 0.2 | 0.3 | 0.3 |
| | Total amount¹⁾ | 0.266 | 0.3495 | 0.3495 | 0.266 | 0.3495 | 0.3495 |
| Insoluble cellulose | Fermentation-derived cellulose preparation | 0.20 | 0.15 | 0.15 | 0.20 | 0.15 | 0.15 |
| | Amount of fermentation-derived cellulose²⁾ | 0.10 | 0.075 | 0.075 | 0.10 | 0.075 | 0.075 |
| Polysaccharide thickner | CMC-Na³⁾ | 0.034 | 0.0255 | 0.0255 | 0.034 | 0.0255 | 0.0255 |
| | Sodium alginate | - | - | 0.15 | - | - | - |
| | calcium chloride | - | - | 0.1 | - | - | - |
| | Soybean polysaccharide | - | - | - | - | - | 1.0 |
| Acid | Citric acid | 0.04 | 0.04 | 0.04 | 0.04 | 0.02 | 0.02 |
| Enzyme | Transglutaminase | 1.0 | 1.0 | 1.0 | 1.0 | 1.0 | 1.0 |
| | Kind of transglutaminase | TG-K | TG-K | TG-K Supple | TG-K | TG-K | TG-K |
| lion-exchanged water | | remaining | remaining | remaining | remaining | remaining | remaining |
| Sum | | 100 | 100 | 100 | 100 | 100 | 100 |

| | | | | | | | |
|---|---|---|---|---|---|---|---|
| 1) Total amount including xanthan gum contained in the fermentation-derived cellulose preparation 2) Amount of xanthan gum contained in the fermentation-derived cellulose preparation 3) Amount of CMC-Na contained in the fermentation-derived cellulose preparation | | | | | | | |

**[Table 2]**

| (Parts by mass) | | Example 7 | Example 8 | Example 9 | Example 10 | Example 11 | Example 12 | Example 13 |
|---|---|---|---|---|---|---|---|---|
| Gelatin | Amount | 5 | 5 | 10 | 7.5 | 3.75 | 5 | 5 |
| | Kind | P-203 | P-203 | P-203 | P-203 | P-203 | P-204 | P-204 |
| Xanthan gum | Amount | 0.3 | 0.3 | 0.3 | 0.2 | 0.2 | 0.3 | 0.3 |
| | Total amount¹⁾ | 0.345 | 0.345 | 0.345 | 0.26 | 0.26 | 0.3 | 0.3 |
| Insoluble cellulose | Fermentation-derived cellulose preparation | 0.15 | 0.15 | 0.15 | 0.20 | 0.20 | - | - |
| | Amount of fermentation-derived cellulose²⁾ | 0.075 | 0.075 | 0.075 | 0.10 | 0.10 | - | - |
| | Citrus fiber | - | - | - | - | - | - | 0.15 |
| Polysaccharide thickner | CMC-Na³⁾ | 0.03 | 0.03 | 0.03 | 0.04 | 0.04 | - | - |
| | Mannan | 0.15 | - | - | - | - | - | - |
| | Agar | - | 0.15 | - | - | - | - | - |
| Other component | Wheat protein decomposition product | - | - | 2 | - | - | - | - |
| Acid | Citric acid | 0.04 | 0.04 | 0.02 | 0.04 | 0.04 | 0.02 | 0.02 |
| Enzyme | Transglutaminase | 1.0 | 1.0 | 1.0 | 1.0 | 1.0 | 1.0 | 1.0 |
| | Kind of transglutaminase | TG-K Supple | TG-K Supple | TG-K | TG-K | TG-K | TG-K | TG-K |
| lion-exchanged water | | remaining | remaining | remaining | remaining | remaining | remaining | remaining |
| Sum | | 100 | 100 | 100 | 100 | 100 | 100 | 100 |

| | | | | | | | | |
|---|---|---|---|---|---|---|---|---|
| 1) Total amount including xanthan gum contained in the fermentation-derived cellulose preparation 2) Amount of xanthan gum contained in the fermentation-derived cellulose preparation 3) Amount of CMC-Na contained in the fermentation-derived cellulose preparation | | | | | | | | |

**[Table 3]**

| (Parts by mass) | | Example 14 | Example 15 | Example 16 | Example 17 | Example 18 | Example 19 |
|---|---|---|---|---|---|---|---|
| Gelatin | Amount | 5 | 5 | 5 | 5 | 5 | 5 |
| | Kind | P-204 | P-205 | P-206 | P-204 | P-204 | P-204 |
| Xanthan gum | Amount | 0.3 | 0.3 | 0.3 | 0.3 | 0.3 | 0.3 |
| | Total amount¹⁾ | 0.3 | 0.345 | 0.345 | 0.345 | 0.345 | 0.345 |
| Insoluble cellulose | Fermentation-derived cellulose preparation | - | 0.15 | 0.15 | 0.15 | 0.15 | 0.15 |
| | Amount of fermentation-derived cellulose²⁾ | - | 0.075 | 0.075 | 0.075 | 0.075 | 0.075 |
| | Microcrystalline cellulose | 0.15 | - | - | - | - | - |
| Polysaccharide thickner | CMC-Na³⁾ | - | 0.03 | 0.03 | 0.03 | 0.03 | 0.03 |
| | Soybean polysaccharide | - | - | - | - | - | 1 |
| | Gadi gum | - | - | - | - | 0.5 | - |
| Other component | Wheat protein decomposition product | - | - | - | - | - | 0.5 |
| Acid | Citric acid | 0.02 | 0.02 | 0.02 | 0.02 | 0.02 | 0.02 |
| Enzyme | Transglutaminase | 1.0 | 1.0 | 1.0 | - | 1.0 | 1.0 |
| | Kind of transglutaminase | TG-K Supple | TG-K | TG-K | - | TG-K | TG-K |
| | Laccase | - | - | - | 1.0 | - | - |
| | Kind of Laccase | - | - | - | Laccase | - | - |
| lion-exchanged water | | remaining | remaining | remaining | remaining | remaining | remaining |
| Sum | | 100 | 100 | 100 | 100 | 100 | 100 |

| | | | | | | | |
|---|---|---|---|---|---|---|---|
| 1) Total amount including xanthan gum contained in the fermentation-derived cellulose preparation 2) Amount of xanthan gum contained in the fermentation-derived cellulose preparation 3) Amount of CMC-Na contained in the fermentation-derived cellulose preparation | | | | | | | |

As each component listed in Tables 1 to 3, the following is used:
Gelatin: SunSupport (registered trademark) P-203 (porcine origin, acid treatment) (San-Ei Gen F.F.I., Inc.); SunSupport (registered trademark) P-204 (porcine origin, acid treatment) (San-Ei Gen F.F.I., Inc.); Sunsupport (registered trademark) P-205 (fish origin, acid treatment) (San-Ei Gen F.F.I., Inc.); and Sunsupport (registered trademark) P-206 (bovine origin, acid treatment) (San-Ei Gen F.F.I., Inc.)
Xanthan gum: Sunsupport (registered trademark) P-207 (San-Ei Gen F.F.I., Inc.)
fermentation-derived cellulose preparation: Sunsupport (registered trademark) P-212 (San-Ei Gen F.F.I., Inc.: containing 50.0% fermentation-derived cellulose, 33.0% xanthan gum, and 17.0% CMC-Na)
Sodium alginate: Sunsupport (registered trademark) P-208 (San-Ei Gen F.F.I., Inc.)
Soybean polysaccharide: Sunsupport (registered trademark) P-214 (San-Ei Gen F.F.I., Inc.)
Mannan: SunSupport (registered trademark) P-209 (San-Ei Gen FFI Co., Ltd.)
Agar: SunSupport (registered trademark) P-210 (San-Ei Gen F.F.I., Inc.)
Wheat protein decomposition product: SunSupport (registered trademark) P-211 (San-Ei Gen F.F.I., Inc.)
Citrus fiber: SunSupport (registered trademark) P-213 (San-Ei Gen F.F.I., Inc.)
Microcrystalline cellulose: Sun Support (registered trademark) P-215 (San-Ei Gen F.F.I., Inc.)
Transglutaminase: Activa (registered trademark) TG-K and Activa (registered trademark) TG-K Supple (Ajinomoto Co., Inc.)
Laccase: Laccase (Amano Enzyme, Inc.)

### Comparative example 1

### PET resin scaffold

BelloCell Chip (CESCO BIOENGINEERING Co. Ltd.) was cut into 5 mm x 5 mm and used. The present PET resin scaffold is hereinafter also referred to as PET scaffold.

### Comparative example 2

### Porous scaffold of gelatin fiber-based material

Genocel (registered trademark) (Nikke Medical Co., Ltd.), a gelatin fiber-based material with a special non-woven structure that can maintain its shape even during cell culture, was used. The porous scaffold of the gelatin fiber-based material is hereinafter also referred to as a gelatin fiber scaffold.

### Test example 1

### Evaluation of adhesive ability and proliferation ability of porous scaffold for cell culture using HEK293T cells

### <Materials>

Cells: HEK293T (human embryonic kidney cells RCB2202; RIKEN)
Medium: 10% FBS 1% PSA I -MEM (10% fetal bovine serum (FBS) (Fuji Film Wako Pure Chemical Corporation), 1% penicillin-streptomycin-amphotericin B (PSA) (Fuji Film Wako Pure Chemical Corporation), I-MEM (food composition medium, IntegriCulture Inc.))
ATP measurement kit CellTiter-Glo@Luminescent Cell Viability Assay (Promega K.K.)
PET resin scaffold of Comparative example 1
Porous scaffold for cell culture of Example 1

### <Cell culture>

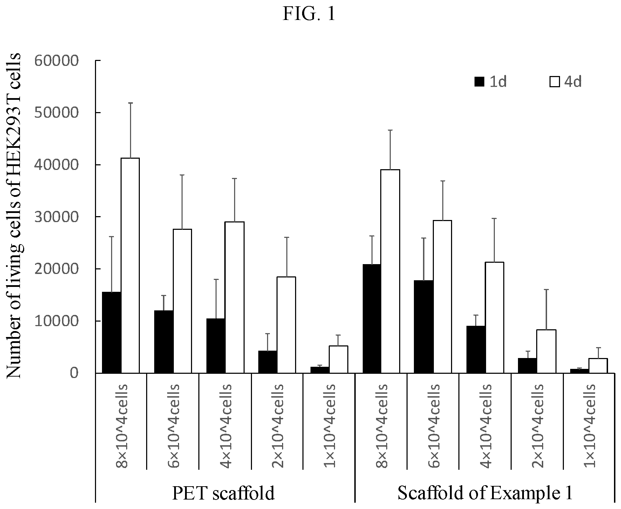

The PET resin scaffold of Comparative example 1 and the porous scaffold for cell culture of Example 1 were cut into 5 mm x 5 mm, and were subjected to autoclaving treatment in a 0.5% CaCl₂ solution at 120°C for 20 minutes. The autoclaved scaffolds were washed with 10% FBS 1% PSA I-MEM, and the CaCl₂ solution was sufficiently replaced with the medium. 4 pieces of the PET resin scaffold of Comparative example 1 were added to each well of a 96-well plate (4 pieces/well). 1 piece of the porous scaffold for cell culture of Example 1 was added (1 piece/well) (n=6). HEK293T cells were prepared in the state of suspension in 10% FBS 1% PSA I-MEM (cell number/well as shown in FIG. 1), and 100 µL of the suspension was seeded into each well. After seeding, the plate was statically cultured at 37°C in an environment of 5% CO₂.

### <Measurement of number of living cells after culture by ATP measurement>

The number of living cells was measured after 1 day and 4 days of culture to evaluate the adhesive ability and proliferation ability of the cells.

The number of living cells after culture was measured using CellTiter-Glo@Luminescent Cell Viability Assay according to the protocol attached to the kit. 1 day or 4 days after cell seeding, the scaffolds were transferred to a new 96-well plate, 50 µL of PBS and 50 µL of CellTiter-Glo@Regent were added to the scaffolds, stirred thoroughly with a vortex mixer to lyse the cells, and ATP was extracted. 100 µL of the obtained lysate was transferred to a new 96-well plate, and the amount of luminescence was measured using a multi-plate reader (Glomax (Promega)), thereby measuring the amount of luminescence based on the amount of ATP. The number of living cells was calculated from the amount of luminescence obtained using a calibration curve created from samples with known cell numbers. The measurement results are shown in FIG. 1 (n=6, error bar: standard deviation).

### <Results and Discussion>

As a result of measuring the number of living cells on day 1 and day 4 of culture of HEK293T cells, it was confirmed in the case of HEK293T cells, that the number of living cells after culture increases as the number of seeded cells increases on day 1 of culture, in both cases of the PET resin scaffold of Comparative example 1 used in the BelloCell culture device (CESCO) and the porous scaffold for cell culture of Example 1 (hereinafter also referred to as the scaffold of Example 1). It was indicated that a constant number of cells adhered to the scaffold regardless of the seeding amount. Furthermore, it was confirmed that the number of living cells increased from day 1 to day 4 of culture no matter which scaffold was used (FIG. 1). From this, it was confirmed that the porous scaffold for cell culture of the present invention has adhesive ability and cell proliferation ability for HEK293T cells. Furthermore, for HEK293T cells, it was confirmed that the scaffold developed in this study had cell adhesive ability and cell proliferation ability equivalent to PET scaffold that is already commercially available as a culture scaffold.

### Test example 2

### Evaluation of adhesive ability and proliferation ability of porous scaffold for cell culture using duck liver-derived primary cells

### <Materials>

Cells: Duck liver-derived primary cells (in-house product: cells were isolated from liver tissue obtained by dissecting ducks, and placed in 10% serum 1% PSA I-MEM (10% serum, IntegriCulture Inc.), and cultured at 37°C)
Medium: 10% serum 1% PSA I-MEM (10% serum, IntegriCulture Inc.), 1% penicillin-streptomycin-amphotericin B (PSA) (Fuji Film Wako Pure Chemical Corporation), I-MEM (food composition medium, IntegriCulture Inc.))
ATP measurement kit CellTiter-Glo@Luminescent Cell Viability Assay (Promega K.K.)
PET resin scaffold of Comparative example 1
Porous scaffolds for cell culture of Examples 1 to 6

### <Cell culture>

The PET resin scaffold of Comparative example 1 and the porous scaffolds for cell culture of Examples 1 to 6 were cut into 5 mm x 5 mm, and were subjected to autoclaving treatment in a 0.5% CaCl₂ solution at 120°C for 20 minutes. The autoclaved scaffolds were washed with 10% FBS 1% PSA I-MEM, and the CaCl₂ solution was sufficiently replaced with the medium. 4 pieces of the PET resin scaffold of Comparative example 1 were added to each well of a 96-well plate (4 pieces/well). Each 1 piece of the porous scaffolds for cell culture of Examples 1 to 6 was added (1 piece/well) (n=6). Duck liver-derived primary cells were prepared in the state of suspension in 10% FBS 1% PSA I-MEM (adjusted to 8 x 10⁴ cells/100 µL of cell suspension), and 100 µL of the suspension was seeded into each well. After seeding, the plate was statically cultured at 37°C in an environment of 5% CO₂.

### <Measurement of number of living cells after culture by ATP measurement>

The number of living cells was measured after 1 day and 4 days of culture to evaluate the adhesive ability and proliferation ability of the cells.

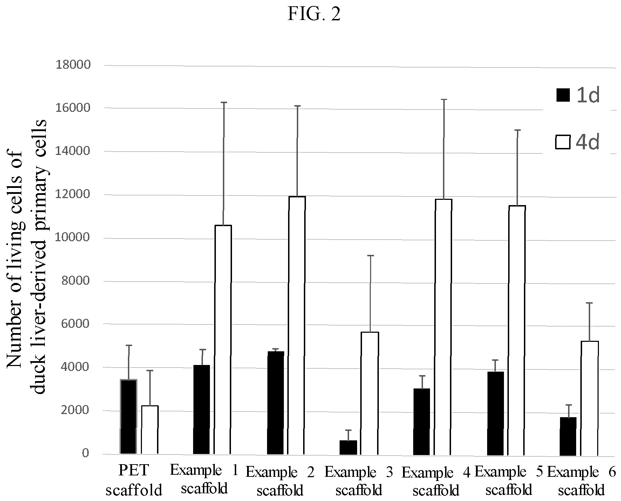

The number of living cells after culture was measured using CellTiter-Glo@Luminescent Cell Viability Assay according to the protocol attached to the kit. 1 day or 4 days after cell seeding, the scaffolds were transferred to a new 96-well plate, 50 µL of PBS and 50 µL of CellTiter-Glo@Regent were added to the scaffolds, stirred thoroughly with a vortex mixer to lyse the cells, and ATP was extracted. 100 µL of the obtained lysate was transferred to a new 96-well plate, and the amount of luminescence was measured using a multi-plate reader (Glomax (Promega)), thereby measuring the amount of luminescence based on the amount of ATP. The number of living cells was calculated from the amount of luminescence obtained using a calibration curve created from samples with known cell numbers. The measurement results are shown in FIG. 2 (n=6, error bar: standard deviation).

### <Results and Discussion>

As a result of counting the number of living cells on day 1 and day 4 of culturing duck liver-derived primary cells, living cells could be confirmed on day 1 and day 4 of culture, for the porous scaffolds for cell culture of Examples 1 to 6 (hereinafter also referred to as Examples 1 to 6 scaffold). Furthermore, an increase in the number of cells was observed from day 1 to day 4 of culture (FIG. 2). Therefore, it was shown that the porous scaffold for cell culture of the present invention has cell adhesive ability and cell proliferation ability for duck liver-derived primary cells.

On the other hand, with respect to the PET resin scaffold of Comparative example 1, although living cells were confirmed, no increase in the number of cells was observed from day 1 to day 4 of culture. Therefore, it is inferred that the combination of cell type, culture scaffold, and culturing method affects the culture results. The possibility was suggested that PET scaffold is not suitable for culturing duck liver-derived primary cells in the 96-well plate as conducted in this study.

On the other hand, cell proliferation was observed for the porous scaffold for cell culture of the present invention, suggesting that it may be more versatile than PET scaffold. The porous scaffold for cell culture of the present invention is based on gelatin which is compounded with xanthan gum or the like to improve the thermal and mechanical stability of the scaffold. Amino acid sequences (RGD sequences) to which proteins such as integrins involved in cell adhesion bind, are contained in gelatin. Accordingly, it is thought that the structure based on gelatin which has high cell adhesive properties, makes it more versatile than PET scaffold.

### Test example 3

### Evaluation of adhesive ability and proliferation ability of porous scaffold for cell culture using duck liver-derived primary cells

Test example 3 is a test using agitation culture using a stirring vessel.

### <Materials>

Cells: Duck liver-derived primary cells (in-house product: cells were isolated from liver tissue obtained by dissecting ducks and placed in 10% serum 1% PSA I-MEM (10% serum, IntegriCulture Inc.), and cultured at 37°C)
Medium: 10% serum 1% PSA I-MEM (10% serum, IntegriCulture Inc.), 1% penicillin-streptomycin-amphotericin B (PSA) (Fuji Film Wako Pure Chemical Corporation), I-MEM (food composition medium, IntegriCulture Inc.))
Cedex Bio (kit reagents: Glucose Bio, Glutame V2 Bio, Lactate Bio, NH3 Bio; Roche Diagnostics Co., Ltd.)
Crystal Violet Dye Nucleus Count Kit (CESCO BIOENGINEERING Co. Ltd)
Corning Microcarrier (Corning International K.K.)
PET resin scaffold of Comparative example 1
Porous scaffolds for cell culture of Examples 1 to 6

### <Cell culture>

The PET resin scaffold of Comparative example 1 and the porous scaffolds for cell culture of Examples 1 to 6 were cut into 5 mm x 5 mm, and were subjected to autoclaving treatment in a 0.5% CaCl₂ solution at 120°C for 20 minutes. The autoclaved scaffolds were washed with 10% FBS 1% PSA I-MEM, and the CaCl₂ solution was sufficiently replaced with the medium. The PET resin scaffold of Comparative example 1 were placed in a stirring culture system at 9 pieces/culture vessel. The porous scaffolds for cell culture of Examples 1 to 6 were placed at 9 pieces/culture vessel, and Corning Microcarriers were placed at 3 mg/culture vessel. Duck liver-derived primary cells were suspended in 10% serum 1% PSA I-MEM (5 x 10⁶ cells), and the cells were seeded in the vessels (5 x 10⁶ cells (liquid volume 5 mL)/culture vessel). After seeding, culture was performed at 37°C 5% CO₂ for 9 days, while the solution was stirred at 5 to 120 rpm.

### <Evaluation of cell adhesive ability and proliferation ability by measuring total cell number after culture>

The total cell number was measured 1 day and 9 days after culture to evaluate cell adhesive ability and proliferation ability.

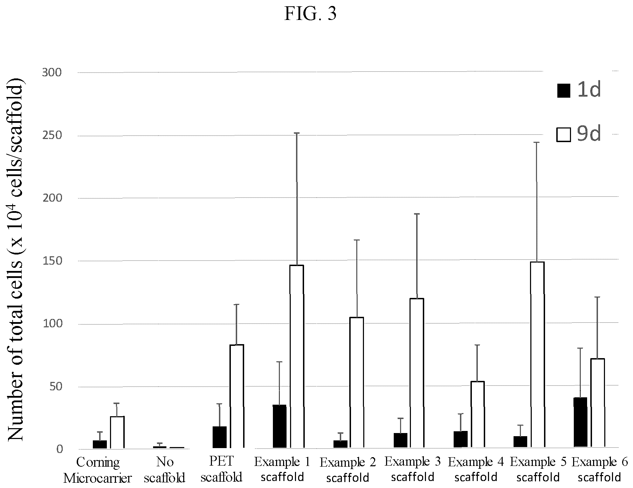

The total cell number after culture was measured using Crystal Violet Dye Nucleus Count Kit, and the measurement procedure was performed according to the protocol attached to the kit. Cells were lysed using Crystal Violet Dye, and nuclei were extracted. The number of nuclei contained in the obtained extract was counted, and the number of cells per scaffold was calculated. The measurement results are shown in FIG. 3 (n=3, error bar: standard deviation).

### <Evaluation of metabolic activity by measuring metabolites in the culture solution>

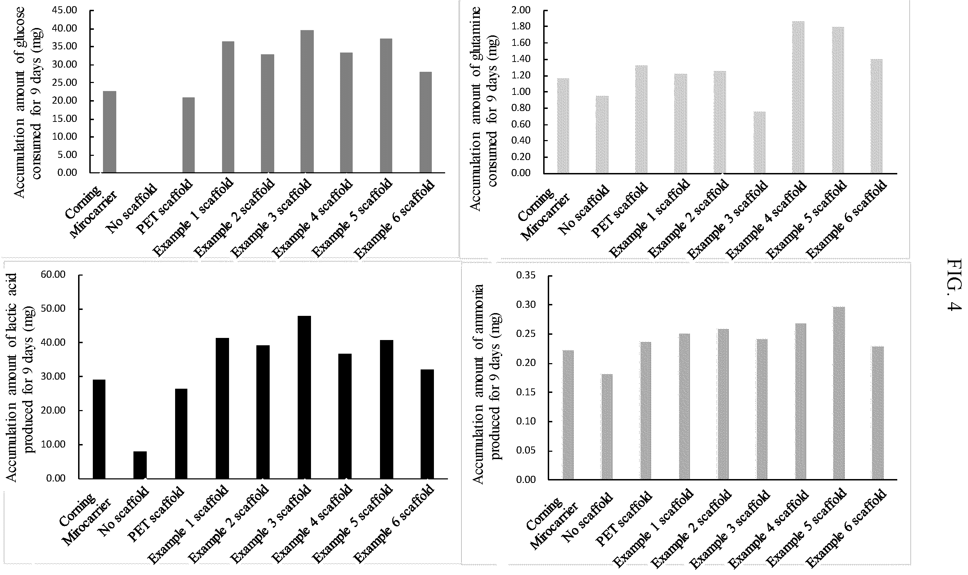

Metabolites in the culture solution were measured using Cedex Bio (n=1). The evaluation of the increase or decrease of metabolites was performed by calculating the consumption amount of the metabolite for measurement of glucose and glutamic acid, and by calculating the integrated value of the production amount for lactic acid and ammonia. The measurement results are shown in FIG. 4.

### <Results and Discussion>

The cell adhesive ability and cell proliferation ability of the porous scaffold for cell culture of the present invention was evaluated using a stirring culture system. As a result of measuring the total cell number on day 1 and day 9 of culture of duck liver-derived primary cells, it was confirmed that the number of cells increased from day 1 to day 9 of culture for the porous scaffold for cell culture of the present invention. (FIG. 3). In addition, Crystal Violet Dye Nucleus Count Kit can only evaluate the total cell number. Accordingly, when the amounts of the consumption and accumulation of metabolites were measured, those that showed an increase in the total cell number were found to have appropriate consumption of glucose and glutamine, and no abnormalities were observed in the amount of lactic acid or ammonia accumulated. Therefore, it was suggested that the increase in total cell number was due to the increase of living cells (FIG. 4). In the test in which no scaffold was added (no scaffold), neither an increase in the total cell number nor consumption of glucose was observed, confirming that duck liver-derived primary cells cannot survive without an appropriate scaffold. These results demonstrate that the culture scaffold developed in this study has cell adhesive ability and cell proliferation ability for duck liver-derived primary cells.

The manufacturer's website reports that Corning Microcarrier has a proven track record in culturing human mesenchymal stem cells. Corning Microcarrier was included in this experiment to potentially serve as a positive control. Although proliferation was observed for the Corning Microcarrier from day 1 to day 9 of culture, the number of cells after 9 days was clearly lower than those of the PET scaffold and the porous scaffold for cell culture of the present invention. This indicates that the porous scaffold for cell culture of the present invention is superior to Corning Microcarrier in duck liver-derived primary cells. In addition, when compared with PET scaffold, the porous scaffold for cell culture of the present invention was confirmed to have the same or higher cell proliferation ability. It was also shown that the porous scaffold for cell culture of the present invention has superiority over Corning Microcarrier and commercially available non-edible PET scaffolds.

### Test example 4

### Evaluation of adhesive ability and proliferation ability of porous scaffold for cell culture using chicken muscle-derived primary cells

Test example 4 is a test using agitation culture in a stirring vessel.

### <Materials>

Cells: Chicken muscle-derived primary cells (in-house product: cells were isolated from liver tissue obtained by dissecting chickens and placed in 10% serum 1% PSA I-MEM (10% serum, IntegriCulture Inc.) and cultured at 37°C)
Medium: 10% serum 1% PSA I-MEM (10% serum, IntegriCulture Inc.), 1% penicillin-streptomycin-amphotericin B (PSA) (Fuji Film Wako Pure Chemical Corporation), I-MEM (food composition medium, IntegriCulture Inc.))
Cedex Bio (kit reagents: Glucose Bio, Glutame V2 Bio, Lactate Bio, NH3 Bio; Roche Diagnostics Co., Ltd.)
Crystal Violet Dye Nucleus Count Kit (CESCO BIOENGINEERING Co. Ltd)
PET resin scaffold of Comparative example 1
Porous scaffold for cell culture of Example 5

### <Cell culture>

The PET resin scaffold of Comparative example 1 and the porous scaffold for cell culture of Example 5 were cut into 5 mm x 5 mm, and autoclaved in 0.5% CaCl₂ solution at 120°C for 20 minutes. The autoclaved scaffolds were washed with 10% serum 1% PSA I-MEM, the CaCl₂ solution was sufficiently replaced with the medium, and the PET resin scaffold of Comparative example 1 were placed in a stirring culture system at 9 pieces/culture vessel. The porous scaffold for cell culture of Example 5 was placed at 9 pieces/culture vessel. Chicken muscle-derived primary cells were suspended in 10% serum 1% PSA I-MEM (5 x 10⁶ cells), and the cells were seeded in the vessel (5 x 10⁶ cells/culture vessel). After seeding, culture was performed at 37°C, 5% CO₂ for 9 days, while stirring the solution at 5 to 120 rpm.

### <Evaluation of cell adhesive ability and proliferation ability by measuring total cell number after culture>

Total cell number was measured 1 day and 9 days after culture to evaluate cell adhesive ability and proliferation ability.

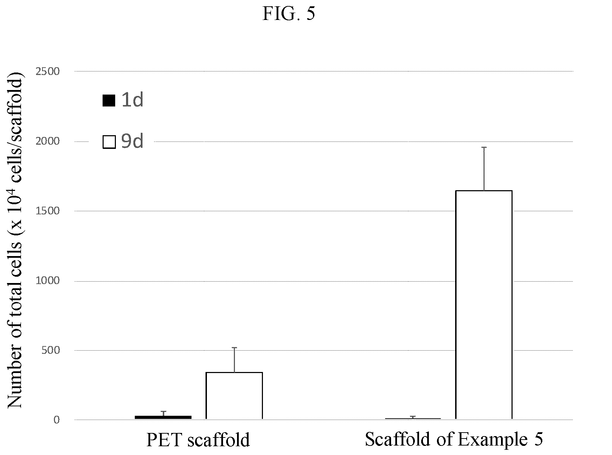

The total cell number after culture was measured using Crystal Violet Dye Nucleus Count Kit, and the measurement procedure was performed according to the protocol attached to the kit. Using Crystal Violet Dye, the cells were thoroughly stirred with a vortex mixer to lyse the cells, and the nuclei were extracted. The number of nuclei contained in the obtained extract was counted, and the number of cells per scaffold was calculated. The measurement results are shown in FIG. 5 (n=3, error bar: standard deviation).

### <Evaluation of metabolic activity by measuring metabolites in the culture solution>

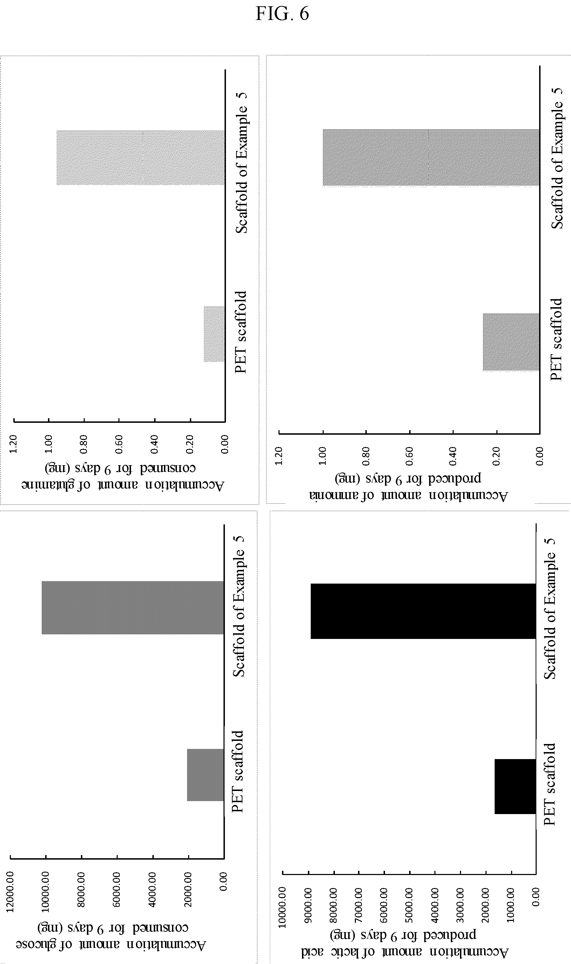

Metabolites in the culture solution were measured using Cedex Bio (n=1). The evaluation of increase or decrease of metabolites was performed by calculating the consumption amount of the metabolites obtained by measurement of glucose and glutamic acid, and by calculating the integrated value of the production amount of lactic acid and ammonia. The measurement results are shown in FIG. 6.

### <Results and Discussion>

The cell adhesive ability and cell proliferation ability of the porous scaffold for cell culture of the present invention were evaluated using a stirring culture system. As a result of measuring the total cell number on day 1 and day 9 of culture of chicken muscle-derived primary cells, it was confirmed that the number of cells significantly increased from day 1 to day 9 of culture for the porous scaffold for cell culture of the present invention (FIG. 5). In addition, when measuring the consumption and accumulation of metabolites, those with an increase of total cell number were observed to have appropriate consumption of glucose and glutamine, and there were also no abnormalities in the accumulation of lactic acid and ammonia (FIG. 6). Therefore, it was indicated that the increase in total cell number was due to an increase in living cells. These results indicate that the scaffold for cell culture developed in this study has cell adhesive ability and cell proliferation ability even for chicken muscle-derived primary cells.

### Test example 5

### Evaluation of adhesive ability and proliferation ability of porous scaffold for cell culture using duck liver-derived primary cells

### <Materials>

Cells: Duck liver-derived primary cells (in-house product: cell pieces were cut out from liver tissue obtained by dissecting ducks, placed in 10% serum 1% PSA I-MEM (10% serum, IntegriCulture Inc.), and cultured at 37°C)
Medium: 10% serum 1% PSA I-MEM (10% serum, IntegriCulture Inc.), 1% penicillin-streptomycin-amphotericin B (PSA) (Fuji Film Wako Pure Chemical Corporation), I-MEM (food composition medium, IntegriCulture Inc.))
Cell Culture Plate Multiwell Plate, with Cover 24 Wells (Corning)
Crystal Violet Dye Nucleus Count Kit (CESCO BIOENGINEERING Co. Ltd)
PET resin scaffold of Comparative example 1
Porous scaffolds for cell culture of Examples 5 and 13 to 19

### <Cell culture>

The PET resin scaffold of Comparative example 1 and the porous scaffolds for cell culture of Examples 5 and 13 to 19 were cut into 5 mm x 5 mm, and immersed in ion exchange water and subjected to autoclaving treatment at 121°C for 20 minutes. The autoclaving-sterilized scaffold was washed with 10% serum 1% PSA I-MEM, and the medium was sufficiently replaced. The PET resin scaffold of Comparative example 1 and the porous scaffolds for cell culture of Examples 5 and 13 to 19 were placed in each well of Cell Culture Plate Multiwell Plate, with Cover 24 Wells at 3 pieces/well. Duck liver-derived primary cells were seeded in 10% serum 1% PSA I-MEM at a density of 2.4 x 10⁵ cells/well (liquid volume: 1 mL/well). Culture was carried out at 37°C in an environment of 5% CO₂ for 1 or 7 days. Medium exchange was conducted after 1 day and 4 days by removing the entire amount of the medium and adding a fresh medium.

### <Evaluation of cell adhesive ability and proliferation ability by measuring total cell number after culture>

Cell adhesive ability and proliferation ability were evaluated by measuring the total cell number 1 day and 7 days after culture.

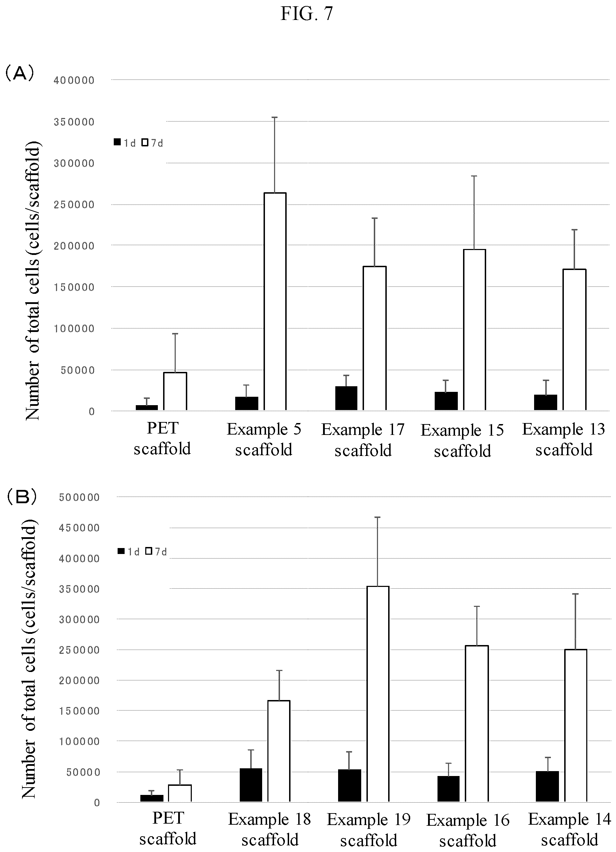

The total cell number after culture was measured using Crystal Violet Dye Nucleus Count Kit, and the measurement procedure was performed according to the protocol attached to the kit. Cell fluid was lysed using Crystal Violet Dye, and nuclei were extracted. The number of nuclei contained in the obtained extract was counted, and the total cell number per scaffold was calculated. The calculated total cell number is shown in FIG. 7 (n=6, error bar: standard deviation).

### <Results and Discussion>

As can be seen from FIG. 7, when compared with the increase in cell adhesion and total cell number on the PET resin scaffold of Comparative example 1 from day 1 to day 7 of culture, both the increase in the cell adhesion and the total cell number on days 1 to 7 of culture by the porous scaffolds for cell culture of Examples 5 and 13 to 19 (hereinafter also referred to as scaffolds of Examples 5 and 13 to 19, respectively) were significant.

Specifically, it was shown that the use of citrus fiber and microcrystalline cellulose as insoluble cellulose also promoted equivalent cell adhesive ability and proliferation ability (Examples 13 and 14). Furthermore, it was shown that the use of fish-derived gelatin and bovine-derived gelatin promoted equivalent cell adhesive ability and proliferation ability (Examples 15 and 16). Furthermore, it was shown that the use of laccase as an enzyme also promoted equivalent cell adhesive ability and proliferation ability (Example 17). It was shown that the use of gum ghatti as the polysaccharide thickener or the use of other components (protein decomposition product) promoted equivalent cell adhesive ability and proliferation ability (Examples 18 and 19).

### Test example 6

### Evaluation of adhesive ability and proliferation ability of porous scaffolds for cell culture using various established cell lines and bovine-derived primary cells

### <Materials>

Cells: RL34 cells (rat-derived liver epithelial cell line), HepG2 cells ( human liver cancer-derived cell line); C2C12 cells (mouse skeletal muscle-derived myoblast cell line); Porcine skeletal muscle satellite cell-derived cell line; Bovine longissimus muscle-derived primary cell line (in-house product: cell pieces were cut out from longissimus muscle obtained from bovine dissection, placed in 10% serum 1% PSA I-MEM medium (IntegriCulture Inc.), and cultured at 37°C); Bovine intermuscular fat-derived primary cells (in-house product: cells were isolated from intermuscular adipose tissue obtained by bovine dissection, placed in 10% serum 1% PSA I-MEM medium (IntegriCulture Inc.), and cultured at 37°C); Bovine biceps muscle-derived primary cells (in-house product: cells were isolated from biceps muscle tissue obtained through dissection of a cow, placed in 10% serum 1% PSA I-MEM medium (IntegriCulture Inc.), and cultured at 37°C)
Medium: 10% FBS (SERENA Europe GmbH), 1% penicillin-streptomycin-amphotericin B (PSA) (Fuji Film Wako Pure Chemical Corporation), D-MEM medium (D-MEM (High Glucose) with L-Glutamine, Phenol Red and Sodium Pyruvate) (Fuji Film Wako Pure Chemical Corporation)
Cell Culture Plate Multiwell Plate, with Cover 24 Wells (Corning)
Crystal Violet Dye Nucleus Count Kit (CESCO BIOENGINEERING Co. Ltd)
PET resin scaffold of Comparative example 1
Porous scaffold of gelatin fiber-based material in Comparative example 2
Porous scaffold for cell culture in Example 5

### <Cell culture>

The PET resin scaffold of Comparative example 1 and the porous scaffold for cell culture of Example 5 were cut into 5 mm x 5 mm, and subjected to autoclaving treatment at 121°C for 20 minutes while immersed in ion-exchanged water. The autoclaving-sterilized scaffold was washed with 10% FBS 1% PSA D-MEM, the medium was sufficiently replaced, and the PET resin scaffold of Comparative example 1, the gelatin fiber-based material porous scaffold of Comparative example 2, and the porous scaffold for cell culture of Example 5 were placed in each well of Cell Culture Plate Multiwell Plate, with Cover 24 Wells, at 3 pieces/well. The various cells used in the test were prepared in a suspended state in 10% FBS 1% PSA D-MEM and seeded at 2.4 x 10⁵ cells/well (liquid volume: 1 mL/well). Culture was carried out at 37°C in an environment of 5% CO₂ for 1 day or 7 days. Medium exchange was conducted after 1 day and 4 days by removing the entire amount of the medium and adding a fresh medium.

### <Evaluation of cell adhesive ability and proliferation ability by measuring total cell number after culture>

Total cell number was measured 1 day and 7 days after culture to evaluate cell adhesive ability and proliferation ability.

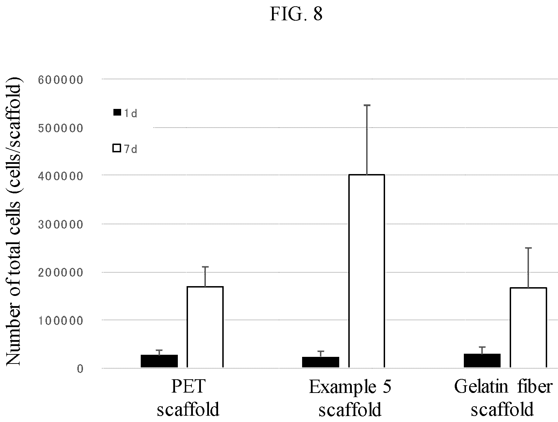

The total cell number after culture was measured using Crystal Violet Dye Nucleus Count Kit, and the measurement procedure was performed according to the protocol attached to the kit. Cells were lysed using Crystal Violet Dye, and nuclei were extracted. The number of nuclei contained in the obtained extract was counted, and the total cell number per scaffold was calculated. The calculated total cell numbers are shown in FIGs. 8 to 10 (n=6, error bar: standard deviation).

### <Results and Discussion>

FIG. 8 shows the results of tests on adhesive ability and proliferation ability of RL34 cells (rat-derived liver epithelial cell line) using the PET resin scaffold of Comparative example 1, the gelatin fiber-based material porous scaffold of Comparative example 2 and the porous scaffold for cell culture of Example 5. As can be seen from FIG. 8, the PET resin scaffold of Comparative example 1 and the gelatin fiber-based material porous scaffold of Comparative example 2 had equivalent cell adhesive ability and proliferation ability, but the porous scaffold for cell culture of Example 5 showed remarkable cell adhesive ability and proliferation ability.

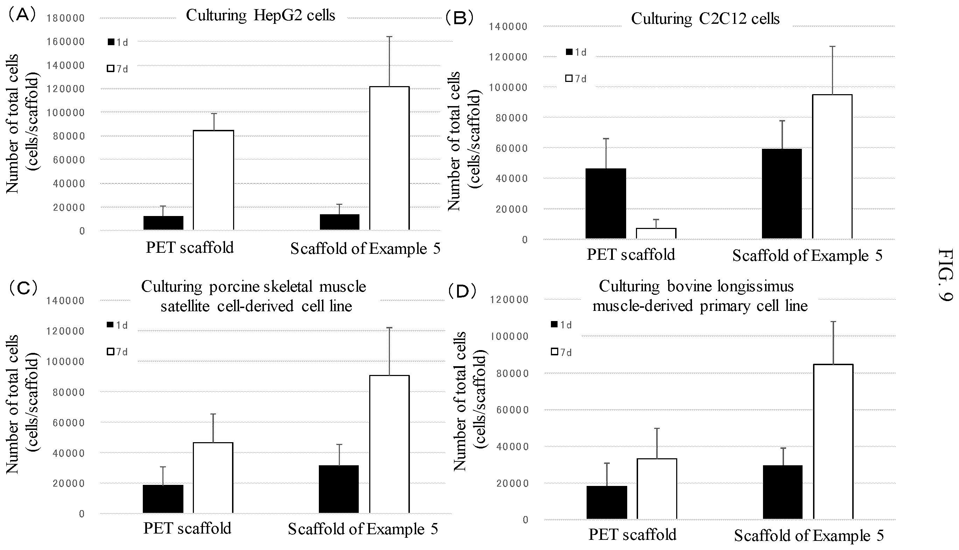

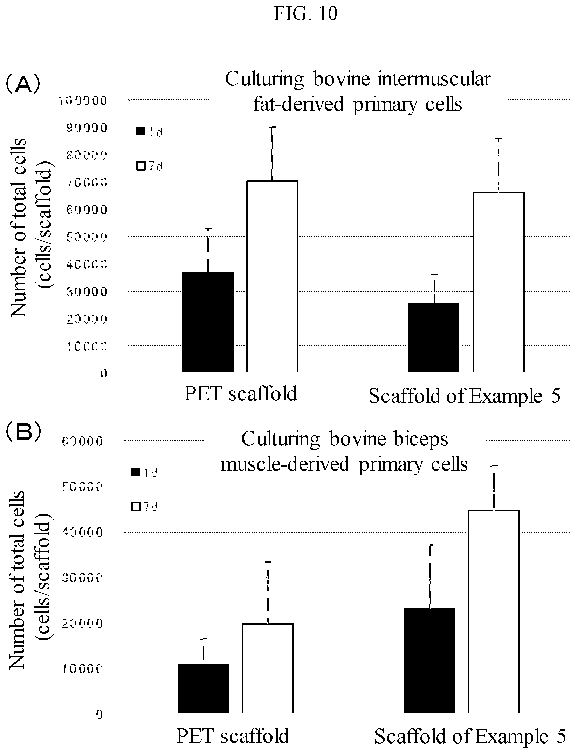

FIGs. 9 and 10 show the results of comparative experiments of the PET resin scaffold of Comparative example 1 and the porous scaffold for cell culture of Example 5 using various established cell lines and bovine-derived primary cells. In both experiments with established cell lines and bovine-derived primary cells, the porous scaffold for cell culture of Example 5 showed the increase in cell adhesive ability and proliferation ability that was equivalent to or superior to the PET resin scaffold of Comparative example 1.

### Test example 7

### Evaluation of shape comparison of porous scaffold for cell culture using duck liver-derived primary cells

### <Materials>

Cells: Duck liver-derived primary cells (in-house product: cell pieces were cut out from liver tissue obtained by dissecting ducks, placed in 10% serum 1% PSA I-MEM (10% serum, IntegriCulture Inc.), and cultured at 37°C)
Medium: 10% serum 1% PSA I-MEM (10% serum, IntegriCulture Inc.), 1% penicillin-streptomycin-amphotericin B (PSA) (Fuji Film Wako Pure Chemical Corporation), I-MEM (food composition medium, IntegriCulture Inc.))
Crystal Violet Dye Nucleus Count Kit (CESCO BIOENGINEERING Co., Ltd.) Ltd)
LUNA-II (trademark) automatic cell counter (Aligned Genetics Inc)
Porous scaffold for cell culture of Example 5

### <Cell culture>

The porous scaffold for cell culture of Example 5 was cut into 5 mm x 5 mm, and sterilized using ethylene oxide gas (EOG). The sterilized scaffold was washed with 10% serum and 1% PSA I-MEM, and the medium was sufficiently replaced, and 2.5 g (dry weight) of the scaffold of Example 5 cut into 5 mm x 5 mm and the scaffold of Example 5 obtained by pulverizing step (pulverized to 1-2 mm) were respectively placed in a 500 mL vessel. The various cells used in the test were prepared in a suspended state in 10% serum 1% PSA I-MEM and seeded at 4.2 x 10⁷ cells/vessel (liquid volume: 200 mL/container). Culture was carried out at 37°C in an environment of 5% CO₂ for 1 day or 15 days. Medium exchange was conducted every day by removing the entire amount of the medium and adding a fresh medium.

### <Evaluation of cell adhesive ability and proliferation ability by measuring total cell number after culture>

The total cell number after 15 days of culture was evaluated using a LUNA-II (trademark) automatic cell counter.

After 15 days of culture, the cells were peeled off from the scaffold and the cell suspension was extracted. The number of cells contained in the obtained extract was counted, and the total cell number per scaffold was calculated.

### <Results and Discussion>

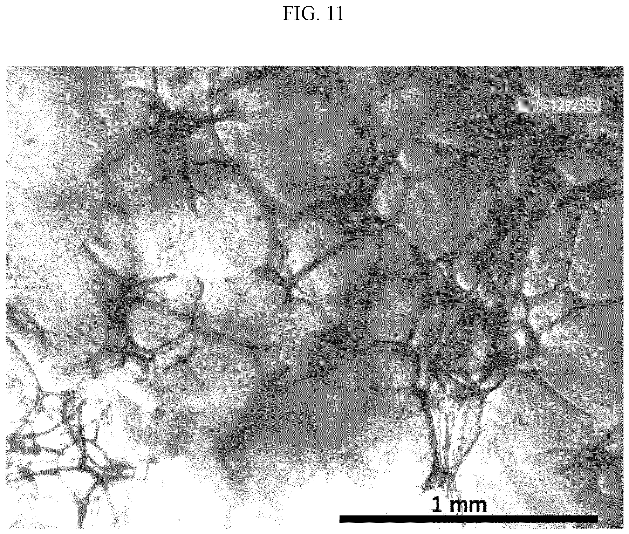

FIG. 11 shows an image of the porous scaffold for cell culture of Example 5 obtained by pulverizing step. This image showed that the 1-2 mm scaffold maintained its porous structure even after pulverizing step. Therefore, the pulverizing step is a step that simply reduces the size of the porous scaffold while maintaining the perforated structure and surface structure. Since the surface area of a porous scaffold increases as it becomes smaller, the pulverizing step also results in an increase of the surface area of the porous scaffold of the same mass. This can also be assumed to increase the area where cells can be cultured.

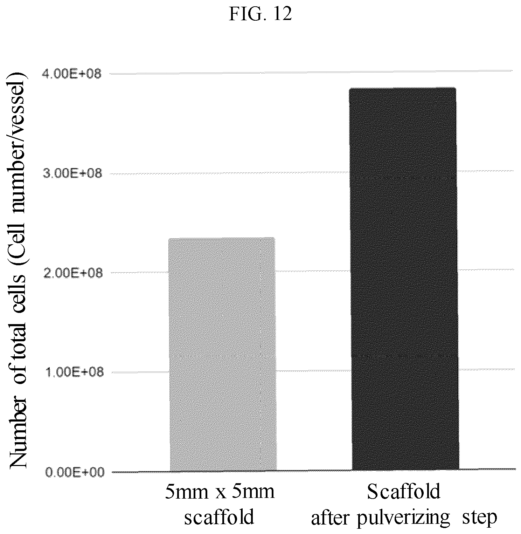

FIG. 12 shows the shape comparison results of the porous scaffold for cell culture of Example 5 using duck liver-derived cells. As can be seen from FIG. 12, it was shown that more cells could be cultured using the scaffold obtained by pulverizing step than when using the scaffold cut into 5 mm x 5 mm. From FIG. 11, it is assumed that in the case of the porous scaffold obtained by pulverizing step, the surface area increases compared to the porous scaffold of the same mass that has not been pulverized by pulverizing step, and the surface area on which cells can be cultured increases. The result of FIG. 12 are considered to be a result expected from FIG. 11.

The embodiments and examples disclosed herein are illustrative in all respects and are not restrictive. The scope of the present invention is indicated by the claims rather than the above description, and includes all changes within the meaning and scope equivalent to the claims.

### ADVANTAGE OF THE INVENTION

The present invention provides a porous scaffold for cell culture which does not deteriorate even after sterilization such as autoclaving, and is capable of culturing various cells; and a production method thereof. Furthermore, the present invention can provide a food composition in which edible cultured cells are attached to a porous scaffold for cell culture, and a production method thereof.

## Claims

1. A porous scaffold for cell culture comprising a crosslinked aggregate of gelatin and xanthan gum or an analog thereof, wherein the shape of the porous scaffold for cell culture is not changed during heat sterilization.

2. The porous scaffold for cell culture according to claim 1, wherein the crosslinked aggregate further comprises insoluble cellulose.

3. The porous scaffold for cell culture according to claim 2, wherein the insoluble cellulose is fermentation-derived cellulose, citrus fiber, or microcrystalline cellulose.

4. A method of producing a porous scaffold for cell culture, comprising:
a mixing and foaming step of mixing and foaming gelatin and xanthan gum or an analog thereof; and
a crosslinking step of crosslinking gelatin and xanthan gum or an analog thereof in the obtained foam.

5. The production method according to claim 4, further comprising a drying step of drying the product obtained in the crosslinking step.

6. The production method according to claim 4 or 5, wherein in the mixing and foaming step, insoluble cellulose is further added to gelatin and xanthan gum or an analog thereof, and the obtained mixture is foamed.

7. The production method according to any one of claims 4 to 6, wherein in the crosslinking step, crosslinking is performed by enzyme treatment and/or heat treatment.

8. A porous scaffold for cell culture produced by the production method according to any one of claims 4 to 7.

9. A food composition in which edible cultured cells are attached to a porous scaffold for cell culture according to any one of claims 1 to 3 and 8.

10. A method of producing a food composition in which edible cultured cells are attached to a porous scaffold for cell culture according to any one of claims 1 to 3 and 8, comprising a culture step of culturing the edible cells together with the porous scaffold for cell culture.

11. The production method according to claim 10, further comprising a differentiation step of differentiating the edible cultured cells.
